# EUROPEAN PATENT APPLICATION

(11) **EP 4 710 992 A1**
(43) Date of publication of application: **18.03.2026**
(21) Application number: 24200941.3
(22) Date of filing: 17.09.2024
(51) Int. Cl.: A61M 60/174, A61M 60/216, A61M 60/414, A61M 60/515, A61M 60/569, A61M 60/861

(54) **A HEART IMPLANT SYSTEM**

(71) Applicant: Pumpinheart Limited, Blackrock, Co. Dublin A94 W3V1 (IE)
(72) Inventor: Malone, Andrew, Galway, H91 DCH9 (IE); Hameed, Aamir, Galway, H91 DCH9 (IE); Colgan, Darragh, Galway, H91 DCH9 (IE); Hickey, Donald, Galway, H91 DCH9 (IE); Ennett, Garry, Galway, H91 DCH9 (IE)
(74) Representative: Purdylucey Intellectual Property

(57) **Abstract**

A heart implant system (1) comprises a heart implant (2, 20) and a delivery catheter (3) detachably attached to the heart implant to percutaneously deliver the heart implant to a heart (60) of a subject. The heart implant comprises an anchoring assembly (4) comprising an anchoring hub (8) and first petals (9) and second petals (10) hingedly attached to the anchoring hub and adjustable relative to the anchoring hub between a crimped delivery configuration and an uncrimped deployed configuration in which the first petals extend radially outwardly of the anchoring hub to limit distal movement of the implant and the second petals form an expandable conforming cage configured to create a stabilising apposition to a wall of a target locus for the implant.

## Description

### Field of the Invention

The present invention relates to a heart implant system, and in particular a heart implant system to treat heart failure with preserved ejection fraction (HFpEF). The invention also relates to a method of treating heart failure with preserved ejection fraction (HFpEF), and a method of relieving or preventing secondary pulmonary hypertension.

### Background to the Invention

Heart failure is defined as inability of the heart to supply adequate blood to the body. As the demographics suggest, with increasing ageing population, prevalence of heart failure is also increasing. The cardinal manifestations of HF are dyspnoea and fatigue, which may limit exercise tolerance, and fluid retention, which may lead to pulmonary and/ or splanchnic congestion and/or peripheral oedema.

Heart cycle has two phases; a contraction phase when heart pumps the blood to the whole body and the relaxation phase when heart is filled with blood. Heart failure associated with the contraction phase (systole) includes HF with reduced ejection fraction (HFrEF), a condition commonly caused by ischemic heart disease that leads to decrease in stroke volume and cardiac output which results in the activation of neurohormonal response in order to restore the normal cardiac output. Left ventricular assist devices have been developed to assist the heart during the contraction phase, including pump devices designed to assist the pumping of blood into the aorta during diastole (contraction), and contractile devices designed to be implanted between two walls of the left ventricle that reciprocate in a pattern synergistic with the heart rhythm to assist the contraction of the left ventricle during systole. Pump devices for treatment of HFrEF are described in US2016/000983 and US7942804.

Heart disease associated with the relaxation phase of the heart cycle (diastole) includes Heart failure with preserved ejection fraction (HFpEF). HFpEF is a clinical syndrome in which patients have symptoms and signs of HF with normal or near normal left ventricular ejection fraction (LVEF >50 percent). During early diastole phase of the cardiac cycle, the healthy LV acts as a 'vacuum cleaner' that enhances the suction particularly during exercise. In HFpEF, cardiomyocyte stiffness results in the loss of this relaxation enhancement, hence normal LV filling is dependent on high left atrial (LA) pressure to push blood into the LV. This pressure elevation can further cause atrial remodelling and secondary pulmonary hypertension, predisposing patients to develop atrial fibrillation and right ventricular (RV) dysfunction. Each 10 mm Hg increment in pulmonary artery pressure in patients with HFpEF was found to be associated with a 28% increase in 3-year mortality 3. Hence, there is a need to effectively control the death rate from HFpEF.

To date, no pharmacological treatment has yet been shown to reduce morbidity and mortality in patients with HFpEF in randomised clinical trials, mainly due to the pathophysiological heterogeneity of the disease. Current treatment strategies as per the American Heart Association (AHA) and European Society of Cardiology (ESC) focus on treating the comorbidities and use of diuretics to relieve congestion.

Transcatheter left to right interatrial shunt devices (REDUCE LAP-HF-I) have been proposed as a possible treatment for HFpEF, although right ventricular (RV) volume overloading and subsequent RV failure and worsening of secondary pulmonary hypertension may be an issue with these shunt devices.

A left ventricular implantable device that applies direct internal expansion forces to increase the LV volume (CORolla) has also been proposed as a possible treatment for HFpEF. In theory, expansive elements in the CORolla device could be used for the treatment of all forms of HFpEF, however performance of such a device would likely be heavily dependent on the degree of cardiac remodelling, which varies significantly over HFpEF phenotypes.

WO2020/081481 describes an implantable device to treat HFpEF comprising a pump that is attached to the mitral valve and is controlled to pump blood from the left atrium to the left ventricle during the cardiac cycle where the pump operates continuously between a low pumping speed (first current) during myocardial perfusion during diastole and a high pumping speed (second current) during the rest of the cardiac cycle. The constant operation of the pump is required as it is attached to the mitral valve and therefore prevents the mitral valve ever fully closing. Thus, during ventricular systole, when the mitral valve is normally fully closed, it is necessary to actuate the pump at high speed to prevent backflow of blood from the left ventricle into the left atrium. The low pumping speed during diastole is required to prevent compression of the left ventricle during diastole. Attaching the pump via a sewing cuff to the mitral valve is a complicated procedure. In addition, the device has a wide bore meaning that it would not be delivered percutaneously and would require heart surgery.

WO2023/118390 describes an implantable heart pump device for the treatment of HFpEF. The device comprises a blood pump that is delivered percutaneously to the heart inside an access sheath, and anchored in the LV of the heart. A controller then actuates the heart pump during ventricular diastole to pull blood into the LV from the LA when the mitral valve is open, and deactivates the pump during ventricular systole when the mitral valve is open.

It is an objective of the invention to overcome at least one of the above-referenced problems.

### Summary of the Invention

Described herein is a heart implant system comprising:
a heart implant; and
optionally, a delivery catheter detachably attachable to the heart implant to percutaneously deliver the heart implant to a heart of a subject,
wherein the heart implant comprises an anchoring assembly comprising an anchoring hub and first petals and second petals hingedly attached to the anchoring hub and adjustable relative to the anchoring hub between a crimped delivery configuration and an uncrimped deployed configuration in which the first petals extend radially outwardly of the anchoring hub and the second petals form an expandable cage.

Typically, the first petals when deployed are configured to inhibit distal movement of the implant.

Typically, the expandable cage when deployed creates a stabilising apposition to a wall of the heart (e.g. an atrial wall).

In any embodiment, the delivery catheter comprises a delivery arm configured for detachable attachment to the implant (e.g. detachable attachment to the hub of the anchoring assembly) and an outer sheath in which the delivery arm is disposed within and axially movable relative to the outer sheath.

In any embodiment, the outer sheath comprises a distal end configured to receive the anchoring assembly of the heart implant when the anchoring assembly is in a crimped delivery configuration, whereby proximal axial movement of the outer sheath relative to the delivery arm effects deployment of the first and second petals.

In any embodiment, the first petal and second petals are configured for self-deployment when the outer sheath is moved proximally relative to the heart implant.

In any embodiment, the first and second petals are dimensioned for deployment of the first petals before deployment of the second petals.

In any embodiment, each of the first petals upon deployment assumes a configuration comprising a proximal section that extends radially outwardly of the anchoring hub and a distal section that curves proximally of the proximal section.

In any embodiment, each of the second petals upon deployment assumes a curved configuration such that the deployed second petals together form a round or oval conformable cage.

Typically, the conformable cage is configured to conform to a shape of a chamber of the heart, for example a left or right atrium or left or right ventricle, a left atrial appendage.

In any embodiment, the first and second petals are formed from a shape-set alloy material.

In any embodiment, each petal comprises a first strut, a second strut, and a distal apex connecting the first and second struts.

In any embodiment, the first and second struts of each first petal, upon deployment, have proximal parts that diverge towards a mid-point of the first petal and distal parts that converge away from a mid-point and towards the distal apex of the first petal.

In any embodiment, the heart implant comprises:
a second assembly; and
a connector operably connecting the anchoring assembly and the second assembly in a spaced-apart relationship.

In any embodiment, the connector is flexible.

In any embodiment, the anchoring assembly is configured for anchoring in a first chamber of the heart and the connector is configured to locate the second assembly in a second chamber of the heart when the anchoring assembly is anchored in the first chamber of the heart.

In any embodiment, the anchoring assembly is configured for anchoring in a left atrium of the heart and the connector is configured to locate the second assembly in a left ventricle of the heart when the anchoring assembly is anchored in the left atrium.

In any embodiment, the second assembly comprises an impeller of a blood pumping device.

In any embodiment, the anchoring assembly comprises a motor for the blood pumping device, and the connector comprises a conduit to deliver blood from the left atrium to the second assembly in the left ventricle and a drive element operably connecting the motor and the impeller.

In another embodiment, the system comprises a remote module configured to be located remotely to the implanted heart implant (e.g. under the skin), wherein the remote module comprises a motor for the blood pumping device and wherein the impeller and motor are operatively connected by a drive element.

In any embodiment, the drive element is a torque cable.

In any embodiment, the anchoring assembly comprises a housing with a fluid inlet and a fluid outlet, the second assembly comprises a housing with a fluid inlet disposed proximally of the impeller and a fluid outlet disposed distally of the impeller, and the connector comprises a conduit that fluidically connects the fluid outlet of the anchoring assembly and the fluid inlet of the second assembly.

In any embodiment, the anchoring assembly housing and the connector conduit are provided by a single conduit.

In any embodiment, the heart implant system is configured such that the fluid inlet of the anchoring housing is closed by the first petals and/or second petals when the first petals and/or second petals are in the folded delivery configuration and open when first and/or second petals are deployed.

In any embodiment, the impeller comprises two or more impellers disposed on a rotor.

In any embodiment, vanes of a first impeller are disposed out of phase to vanes of a second impeller.

In any embodiment, the heart implant system comprises a controller configured to modify the output parameters of the pump so as to activate and deactivate the pump in a pattern synergistic with a cardiac cycle of the subject comprising activation during ventricular diastole and deactivation during ventricular systole.

In any embodiment, the heart implant system comprises at least one sensor in communication with the controller for detecting one or more parameters associated with the heart of the subject, wherein the controller is configured to modify the output parameters of the pump based on the one or more detected parameters received from the sensor.

In any embodiment, the heart implant system comprises an implantable power unit and a power lead configured to percutaneously electrically couple the implantable power unit and the pump.

Also described is a heart implant comprising:
a blood pumping device comprising a motor, an impeller, and a drive element operably connecting the motor and the impeller;
a proximal anchoring part to anchor the blood pumping device to the heart; and;
a distal part; and
a connector operably connecting the proximal anchoring part and the distal part in a spaced-apart relationship such that when the distal part is located in a first chamber of the heart the proximal anchoring part may be located in a second chamber of the heart.

In any embodiment, the proximal anchoring part comprises the motor, the distal part comprises the impeller, and the drive element comprises a torque cable.

In another embodiment, the system comprises a remote module configured to be located remotely to the implanted heart implant (e.g. under the skin), wherein the remote module comprises a motor for the blood pumping device and wherein the impeller and motor are operatively connected by a drive element.

In any embodiment, the connector is flexible.

In any embodiment, the connector comprises a conduit and the torque cable is disposed within the conduit.

In any embodiment, the proximal anchoring part comprises a housing with a fluid inlet and a fluid outlet, the distal part comprises a housing with a fluid inlet disposed proximally of the impeller and a fluid outlet disposed distally of the impeller, and the connector comprises a conduit that fluidically connects the fluid outlet of the anchoring assembly and the fluid inlet of the distal part.

In any embodiment, the anchoring part comprises an expandable frame adjustable between a contracted delivery configuration and an expanded anchoring configuration dimensioned to anchor the expanded anchoring assembly in a chamber of the heart other than the left ventricle by exerting a radial or outward pressure against the walls of the chamber.

In any embodiment, the anchoring part comprises an anchoring hub and first petals and second petals hingedly attached to the anchoring hub and adjustable relative to the anchoring hub between a crimped delivery configuration and an uncrimped deployed configuration in which the first petals extend radially outwardly of the anchoring hub and the second petals form an expandable cage.

Typically, the first petals when deployed are configured to inhibit distal movement of the implant.

Typically, the expandable cage when deployed creates a stabilising apposition to a wall of the heart (e.g. an atrial wall).

In any embodiment, the heart implant system comprises an implantable power unit and a power lead configured to electrically couple the implantable power unit and the motor.

Also described is an anchoring and stabilising assembly for a heart implant comprising an anchoring hub and first petals and second petals hingedly attached to the anchoring hub and adjustable relative to the anchoring hub between a crimped delivery configuration and an uncrimped deployed configuration in which the first petals extend radially outwardly of the anchoring hub to inhibit distal movement of the implant and the second petals form an expandable cage configured to create a stabilising apposition to a wall of the heart (e.g. an atrial wall)..

In any embodiment, the heart implant system comprises the first petal and second petals are biassed into the uncrimped deployed configuration and are configured for deployment by relative axial movement of the delivery catheter outer sheath and delivery catheter control arm resulting in the implant being exposed out of a distal end of the outer sheath and deployment of the first petals and second petals.

In any embodiment, the heart implant system is configured to fully deploy the first petals before the second petals.

In any embodiment, each of the first petals upon deployment assumes a configuration comprising a proximal section that extends radially outwardly of the anchoring hub and a distal section that curves proximally of the proximal section.

In any embodiment, each of the second petals upon deployment assumes a curved configuration such that the deployed second petals together form a round or oval cage.

In any embodiment, the first and second petals are formed from a shape-set alloy material.

In any embodiment, each petal comprises a first strut, a second strut, and a distal apex connecting the first and second struts.

In any embodiment, the first and second struts of each first petal, upon deployment, have proximal parts that diverge towards a mid-point of the first petal and distal parts that converge away from a mid-point and towards the distal apex of the first petal.

In any embodiment, the anchoring assembly comprises a central tubular housing having a distal end, a proximal end, and a sidewall extending between the distal and proximal ends, in which the distal hub is disposed on the distal end of the housing.

In any embodiment, the proximal end of the housing comprises a chamber configured to receive and store an electrical connection lead.

In any embodiment, the system comprises a sensor to detect heart rate or heart cycle data, and a controller to control the motor, in which the sensor is operably connected to the controller.

In any embodiment, the controller is configured for implantation, typically sub-dermal implantation.

In any embodiment, the system comprises a power supply operably connected to the motor via the controller.

In any embodiment the controller comprises a processor configured to receive cardiac cycle data from the sensor and activate and deactivate the motor in a pattern synergistic with the cardiac cycle including activation during diastole and deactivation during systole.

Also described is a method comprising:
providing a heart implant system according to the disclosure in a delivery configuration;
advancing the heart implant system along a guidewire to a target location in a body of the subject;
deploying the first petals of the anchoring assembly to limit distal movement of the implant relative to the target location;
deploying the second petals of the anchoring assembly to stabilise the implant at the target locus; and
fully detaching the delivery arm of the delivery catheter from the implant.

In any embodiment, the method employs a heart implant system of the disclosure.

In any embodiment, the first petals are fully deployed before the second petals are deployed.

In any embodiment, the system comprises a second assembly spaced apart from the anchoring assembly and a connector comprising a fluidic conduit connecting the anchoring assembly and the second assembly.

In any embodiment, the method comprises pumping blood from the first location to the second assembly through the fluidic conduit.

In any embodiment, the second assembly comprises an impeller, the anchoring assembly comprises a motor, and the implant comprises a torque wire operably connecting the motor and the impeller for rotation thereof, wherein the method comprises actuation of the motor to pump blood.

In any embodiment, the second assembly comprises an impeller, the system comprises a motor located remotely to the implant, and the system comprises a drive element (e.g. a torque wire) operably connecting the motor and the impeller for rotation thereof, wherein the method comprises actuation of the motor to pump blood.

Also described is a method of treating or prevent left side heart failure, especially HFpEF, comprising implanting an implant of the disclosure into the heart of a subject (in need thereof) in which the anchoring assembly is anchored in the left atrium and the second assembly is located in the left ventricle, and actuating the motor of the implant in a pattern synergistic with the cardiac cycle including activation during ventricular diastole and deactivation during ventricular systole.

Other aspects and preferred embodiments of the invention are defined and described in the other claims set out below.

### Brief Description of the Figures

**FIG.** 1 is an illustration of an embodiment of the heart implant system of the invention showing the staged deployment of the heart implant, in which: **FIG. 1A** shows the heart implant during delivery; **FIG. 1B** shows the heart implant system during an initial stage of deployment with the outer sheath of the delivery catheter partially retracted to deploy the first petals and the second petals still retained within the outer sheath; and **FIG. 1C** shows the delivery catheter fully detached from the heart implant and the second petals deployed.
**FIG. 2** is an exploded view a heart implant according to one embodiment of the invention.
**FIG. 3** is a side elevational view of the heart implant of Figure 2 implanted in a heart of a subject with the anchoring assembly anchored in the left atrium and the second assembly containing an impeller disposed in the left ventricle. The anchoring assembly contains a motor which is operably connected to the impeller for rotation thereof by a wire or torque cable..
**FIG. 4** is a detailed perspective view of a heart implant system of the invention in a delivery configuration showing the heart implant attached to a distal end of a delivery catheter and mounted on a guidewire.
**FIG. 5** is a detailed perspective view of a heart implant system of Figure 4 during an initial stage of deployment where the first and second petals are constrained within the outer sheath of the delivery catheter.
**FIG. 6** is a detailed perspective view of a heart implant system of Figure 4 during an intermediate stage of deployment where the first are free of the outer sheath of the delivery catheter and about to self-deploy and second petals are still constrained within the outer sheath of the delivery catheter.
**FIG. 7** is a detailed perspective view of a heart implant system of Figure 4 in a delivery configuration with the delivery catheter removed to illustrate the anchoring assembly in more detail.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or openended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

Additionally, the terms "treatment" or "treating" refers to an intervention (e.g., the use of the device of the invention to assist systole of the left ventricle) which prevents or delays the onset or progression of a disease or reduces (or eradicates) its incidence within a treated population. In this case, the term treatment is used synonymously with the term "prophylaxis".

In the context of treatment and effective amounts as defined above, the term subject (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

"Activation during ventricular diastole and deactivation during ventricular systole" means that the blood pumping device is activated during at least a part of ventricular diastole (usually at least 50%, 60%, 70%, 80%, 90% or 100% of ventricular diastole) and the blood pumping device is deactivated during ventricular systole. For example, the blood pumping device may be deactivated when system detects closure of the aortic valve (during ventricular systole) and may be activated when the system the opening of the aortic valve (during ventricular filling and subsequent atrial contraction). The blood pumping device may be activated and deactivated at a frequency based on the frequency of the heartbeat detected by, for example, a heart pacing sensor.

The system of the invention may include an inductive charging apparatus. "Inductive charging apparatus" means an apparatus for charging the system, or any device of the system, wirelessly by using any one of electromagnetic field, wireless radio waves or magnetic resonance charging to transfer energy to charge the device. For example, the blood suction device may comprise an inductive charging apparatus.

The system of the disclosure generally comprises a delivery catheter. This usually includes a delivery arm detachably attached to the implant, and an outer sheath. The delivery arm is generally disposed within and axially movable relative to the outer sheath. During deployment, relative movement between the delivery arm and outer sheath exposes the implant proud of a distal end of the outer sheath which allows the first and second petals to deploy. The distal end of the outer sheath may comprise an enlarged lumen section to house the implant during delivery. The system may comprise a control handle operably connected to proximal ends of the delivery arm and outer sheath. The handle may comprise a first actuator to effect relative movement of the outer sheath relative to the delivery arm. The handle may comprise a second actuator to detach a distal end of the delivery arm from the implant.

The system of the invention may be employed to deliver clinically effective functional support to the left ventricle of the heart during the filling phase (diastole). The system increases the flow of blood into the left ventricle of the heart during diastole in which it is implanted and then is deactivated during systole. In one embodiment, the blood pumping device is deactivated during all of ventricular systole. The controller is programmable such that the system can act as an auto-adaptive system that responds to physiologic cues. The system can continuously adapt to specific user requirements. The blood suction device is configured by means of the controller to activate in a pattern synergistic to the natural cycle of the heart to, for example, activate the blood suction device during diastole and deactivate the blood suction device during systole. Thus, as the heart rate of a subject increases, the frequency of activation and deactivation of the blood suction device changes in sync with the heart rate based on signals received from the sensor. In addition, the controller can modify the impeller rotation speed based on physiological cues received from the sensor, for example increase the impeller rotational speed during periods of elevated heartbeat and reduce the impeller rotational speed during periods of reduced heart rate. The controller may comprise a computation device configured to receive data from the sensor, compare the stored with stored reference data, and modify the output parameters of the blood suction device based on the comparison.

The controller may be configured for use outside the heart. Thus, the system may include a control lead that operatively couples the implant within the heart with the controller located outside the body. The control lead may extend through a wall of the heart and through the chest to the external controller. In another embodiment, the control lead may extend percutaneously from the implant through part of the vasculature and out of the body at a suitable location. The controller may be configured to be wearable by a subject. The controller may be connected to a power source, for example a battery. In another embodiment, the controller may be configured for implantation within the heart, as part of the blood suction device, and be configured to receive data from the sensor.

The sensor may be part of the system of the invention, or the system of the invention may be configured for use with a separate sensor. For example, the system may include a sensor configured for implantation in the left ventricle of the heart where it is operatively connected or coupled to the blood suction device. This may be a wire/sensor element configured to extend into contact with a wall of the heart for sensing a parameter of heart contraction/relaxation or configured to contact blood in the left ventricle or another chamber to detect a parameter of the blood (for example pressure or flow rate).

In another embodiment, the sensor is configured for implantation on an external wall of the heart and operatively coupled to the controller, which may be mounted externally of the subject's body.

In any embodiment, the system comprises a graphical user interface which may form part of the controller. The controller may be configured to display on the GUI data relating to the functioning of the system, for example heart rate, blood pressure, frequency of activation of the impeller, rotational speed of the impeller.

In any embodiment, the sensor is configured to detect ejection fraction of the left ventricle. In any embodiment, the controller is configured to compare the detected ejection fraction of the left ventricle with one or more ejection fraction reference values and modify the output parameters of the blood suction device based on the comparison. In any embodiment, the controller is configured to switch off the blood suction device for a period of time when the ejection fraction is detected to be reduced. In any embodiment, the controller is configured to resume operation of the blood suction device when the ejection fraction is detected to be preserved.

The system may further include telemetric components for transmitting and receiving signals relating to the activity of the left ventricle, heart or the activity of the system. Telemetric components may include wireless medical telemetric elements using radio frequency to relay data such as pulse, heart rate, and electrical activity of the heart to the controller.

The controller may be configured to control the operation of the blood suction device either through automatic execution of program instructions in memory and/or upon receiving an external input from a user. The memory component of the system may include ROM and RAM memory. The controller may take the form of a microprocessor.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

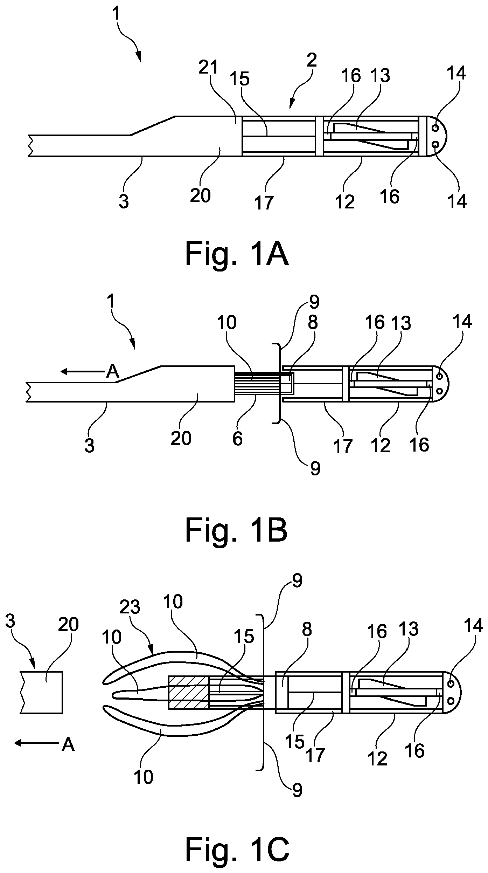

Referring to the drawings and initially to Figures 1A to 1C, a heart implant system (hereafter "system") according to the disclosure is described. The system 1 comprises a heart implant (hereafter "implant") 2 and a delivery catheter 3 comprising an outer sheath 20 and delivery arm (not shown). In this embodiment of the system, the heart implant 2 comprises an anchoring assembly 4 and a second assembly 5 operably connected to the anchoring assembly 4.

The anchoring assembly 4 comprises a housing 6 containing an electric motor 7 and a distal hub 8 comprising first petals 9 and second petals 10. The petals are formed from nitinol and are shape set into the deployed configuration shown in Figure 1C.

The second assembly 5 comprises a cylindrical housing 12 containing an impeller 13 mounted on bearings 16 for rotation within the housing. A distal end of the cylindrical housing 12 has fluid outlets 14. The impeller 13 is operatively coupled to the electric motor 7 by a torque wire 15. The anchoring assembly 4 and second assembly 5 are connected, in this case by a fluidic conduit 17, which contains the torque wire 15 and provides fluidic communication between left atrium and the cylindrical housing to allow blood to be pumped from the left atrium into the left ventricle during actuation of the motor. The fluidic conduit 17 and torque wire 15 may both be flexible to allow a degree of play between the anchored anchoring assembly 4 and second assembly 5 when the implant is implanted in a subject heart, which has been found to provide for more secure and robust anchoring the implant in the heart.

Figure 1A shows the system in a delivery configuration. The anchoring assembly 4 is fully contained within a distal end 21 of an outer sheath 20 forming part of the delivery catheter 3. In this delivery configuration, the first petals 9 and the second petals 10 are retained in a tensioned crimped configuration by the outer sheath, where the petals lie substantially parallel to a longitudinal axis of the delivery catheter 3. The outer sheath 20 and anchoring assembly 4 are dimensioned for a friction fit, fluid tight, attachment.

Figure 1B shows the system during an initial stage of deployment with the outer sheath 20 of the delivery catheter 3 partially retracted to fully expose and release the first petals 9 which self-deploy into a radial outward configuration. At this stage, the second petals 10, which are longer than the first petals, are not fully exposed and are constrained into the crimped configuration by the outer sheath 20.

Figure 1C shows the delivery catheter 3 fully detached from the heart implant 2 and the second petals 10 fully exposed and deployed into their curved uncrimped configuration. The plurality of second petals 10 together forms an oval cage 23 disposed proximally of the deployed first petals 9.

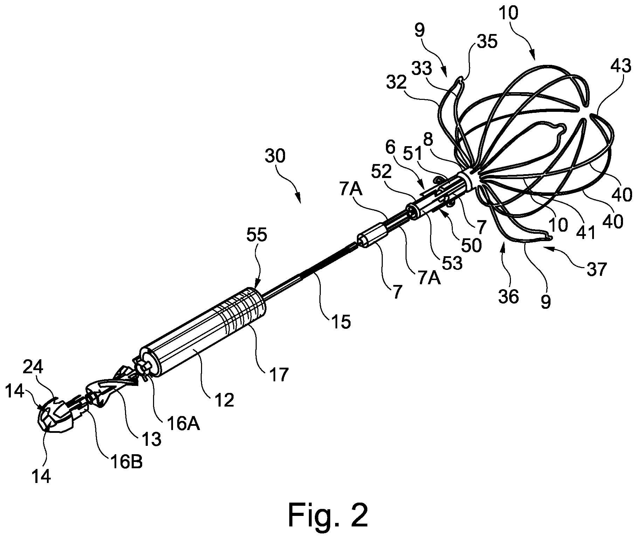

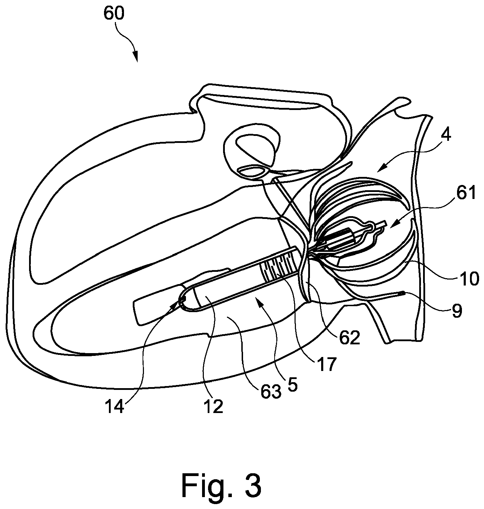

The system will now be described in more detail with reference to Figures 2 and 3.

Referring initially to Figure 2, an embodiment of an implant is described in which parts described with reference to Figure 1 are assigned the same reference numerals. The implant, indicated generally by the reference numeral 30, and shown in an exploded view, comprises (from right to left) anchoring assembly with anchoring hub 8 and deployed first petals 9 and second petals 10, anchoring assembly housing 6, electric motor 7 and motor electrical leads 7A, torque wire 15, fluidic conduit 17, second assembly housing 12, first impeller bearing 16A, impeller 13, and second impeller bearing 16B. The second impeller bearing 16B comprises an end cap 24 for a distal end of the housing 12 having fluid outlets 14. In this embodiment, the second assembly housing 12 and fluidic conduit 17 are provided by a single hollow cylindrical housing.

In more details, the four first petals 9 are arranged around a circumference of the distal hub 8. The hub 8 is a cylindrical ring. Each first petal 9 comprises two struts 32 having proximal ends 33 connected to the distal hub 8 and distal ends connected at a first petal apex 35. The first petals 9 are configured to deploy into radially outward configuration having a proximal section 36 and a distal section 37 which sweeps rearwardly of the proximal section (similar to winglets on a wing) which in this embodiment matches the anatomy of the left atrium. The struts diverge from a proximal end towards a midpoint and then converge towards the first apex 35. The first petals 9 on deployment form an annulus anchor and allow the implant to be positioned correctly with the anchoring assembly in a first chamber of the heart (e.g. the left atrium) and the second assembly in the second chamber (e.g. the left ventricle).

The four second petals 10 are also arranged around a circumference of the distal hub 8, interspersed between the first petals 9. Each second petal 10 comprises two struts 40 having proximal ends 41 connected to the distal hub 8 and distal ends connected at a second petal apex 43. Each of the second petals 10 is configured to deploy into curved or arched configuration such that the upon deployment the four second petals 10 form an oval cage 23. The struts 40 diverge from a proximal end towards a strut midpoint and then converge towards the second apex 43. The deployed oval cage is dimensioned to anchor the implant 30 in the first chamber by exerting pressure against the walls of the chamber. In the implant illustrated, the annulus anchor and oval cage are dimensioned for deployment and anchoring within the left atrium of the heart.

The struts of the first petals 9 are about 50% of the length of the struts of the second petals (but may be 30% to 70%). This means that upon retraction of the outer sheath (not shown) the first petals 9 deploy prior to the deployment of the second petals 10. This allows a user position the implant abutting the mitral valve to prevent further distal movement of the implant, and then once the user is satisfied with the axial position of the implant they can then deploy the second petals 10 to prevent any lateral movement of the implant.

The anchoring assembly housing 6 is a hollow cylinder 50 with a proximal part 51 to house the motor 7 and a distal part 52 with sidewall openings 53 and an open distal end 54. In a delivery configuration, the folded petals 9, 10 surround and close the sidewall openings 53.

On assembly, the motor 7 is located in the proximal part 51 of the housing 6 and the distal hub 8 is attached to the distal part 52 of the housing 6. The distal hub is then inserted into an open proximal end 55 of the fluidic conduit 17, thus providing fluidic communication between the sidewall openings 53 of the anchoring assembly housing 6 and the impeller 13 disposed in the second assembly 5.

Figure 3 illustrates the implant 30 of Figure 2 implanted in the heart 60 of a subject with the anchoring assembly 4 anchored in a left atrium 61 just proximal of a mitral valve 62 and the second assembly 5 located in the left ventricle 63. When the petals 9, 10 are fully deployed and the motor 7 is actuated, the impeller (not shown) rotates to draw blood from the left atrium into the sidewall openings 53 of the anchoring assembly, through the fluidic conduit 17 and second assembly housing 12 and out of the fluid outlets 14 into the left ventricle. Thus, the device can be employed to help the left ventricle fill with blood during a relaxation phase of the cardiac cycle. In addition, the implant obviates the need to locate an anchor in the left ventricle which is problematical due to the vigorous movement of the left ventricle during the cardiac cycle.

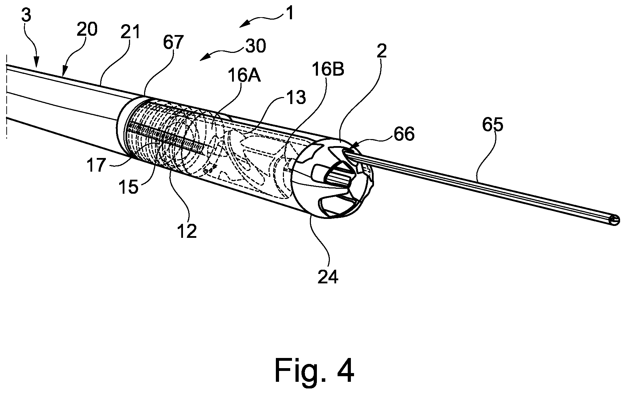

Figures 4 to 7 illustrate the deployment of the implant in which parts described with reference to Figures 2 and 3 employ the same reference numerals.

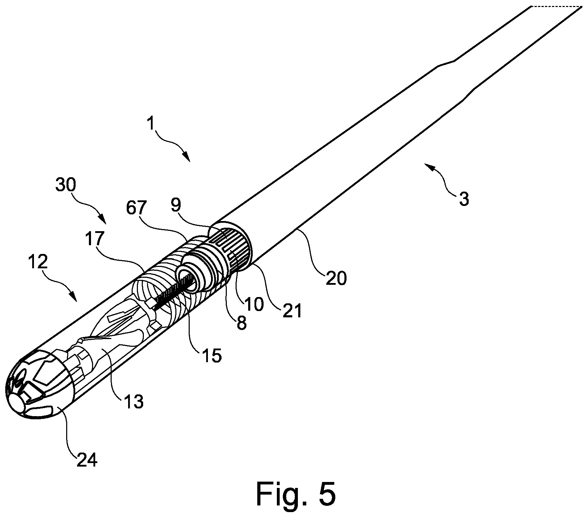

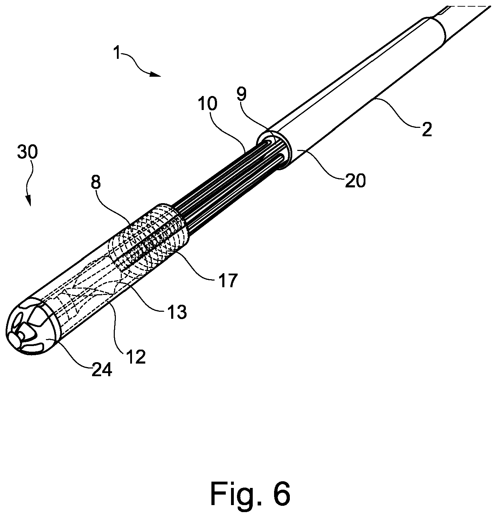

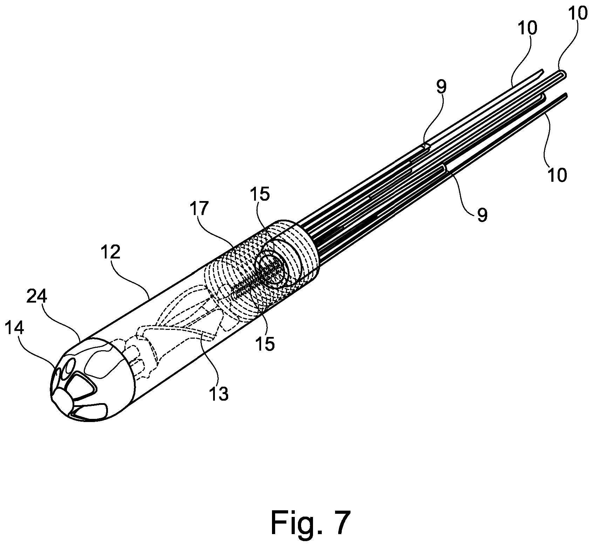

Referring initially to Figure 4, the implant 30 is delivered percutaneously to the heart along a guidewire 65, generally via the femoral artery. The end cap 24 of the second assembly 5 includes an aperture 66 for receiving the guidewire 65, which is located off-centre towards a periphery of the end cap. The implant 30 (specifically, usually the hub of the anchoring assembly) is attached to the delivery arm (not shown) of the delivery catheter 3 during delivery, with the outer sheath 20 of the delivery catheter in a fully advanced position (relative to the implant) abutting a proximal end 67 of the fluidic conduit 17. In this configuration, the anchoring assembly 4 is disposed in a distal end of the outer sheath 20, thus constraining the first and second petals 9, 10 into a crimped tensioned configuration inside the outer sheath (Figure 7 shows the anchoring assembly during delivery with the outer sheath removed for clarity). Referring to Figures 5 and 6, once the interventionalist is satisfied that the second assembly 5 is located in the left ventricle and the distal end of the outer sheath 20 is located just inside the left atrium, the delivery catheter 3 is actuated to retract the outer sheath 20 relative to the implant 30 to fully expose the first petals 9 which then deploy. Further retraction of the outer sheath 20 fully exposes the second petals 10 which deploy for form the open cage. In this way, the first petals 9 are deployed first to form the annulus anchoring structure (which limits further distal movement of the implant), and then the second petals 10 are deployed to form the atrial stabilising structure (which securely anchors the implant to the wall of the left atrium. Once fully deployed, the delivery arm is detached from implant and the delivery arm and outer sheath 30 are retracted to leave the implant in-situ.

The system and implant of the disclosure provides a number of advantages. The anchoring system having first petals that limit axial movement and second petals which anchor the device to a chamber of the heart enables the implant to be positioned correctly and, separately, anchored. The configuration of the first and second petals allows a two-stage deployment process where the first petals help position the implant (and limit distal movement) before the second petals are deployed to anchor the implant. The location of the motor in the anchoring assembly and the impeller in the second assembly (and the use of a torque wire/cable connecting the spaced apart motor and impeller) reduces the size of the second assembly. Moreover, the use of a flexible connecting conduit between the second assembly and the anchoring assembly (combined with a flexible torque wire) provides the second assembly with a degree of play relative to the anchored anchoring assembly, which is advantageous when the second assembly is located in a ventricle.

The implant may be employed to anchor an implantable device in a body locus, for example a chamber of a heart, or inside a body lumen such as a vessel. The implant is particularly suitable for anchoring an implant in a mammalian heart, and in particular anchoring an implant in a heart where the anchoring assembly is located in one chamber of the heart and a second assembly (for example a blood pumping device) in a second chamber of the heart. Specific embodiments are implants with an anchoring assembly configured for deployment in an atrium of the heart and a second assembly for locating in a ventricle.

In the embodiments illustrated, the connector (between the anchoring assembly and the second assembly) comprises a conduit for blood, to pass blood from the chamber in which the anchoring assembly is located to the second assembly containing the impeller. In other embodiments, the connector may not require a conduit for blood. Also, in the present embodiment, the housing for the second assembly and the connector (conduit) are provided by a single conduit (a flexible cylindrical tube). It will be appreciated that the fluidic conduit (connector) and the housing for the second assembly may be separate pats configured to fluidically connect together during assembly.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

### Reference Numerals

Heart implant system 1
Heart implant 2, 30
Delivery catheter 3
Anchoring assembly 4
Second assembly 5
Housing (anchoring assembly) 6
Electric motor 7
Electrical leads 7A
Distal hub 8
First petals 9
Second petals 10
Housing (second assembly) 12
Impeller 13
Fluid outlet 14
Torque wire 15
Bearings 16A, 16B
Fluidic conduit 17
Outer sheath 20
Distal end (outer sheath) 21
Oval cage 23
Closure for
Struts (first petal) 32
Proximal end of strut 33
First petal apex 35
Proximal section of first petals 36
Distal section of first petals 37
Struts (second petal) 40
Proximal end of strut 41
Second petal apex 43
Hollow cylinder (anchoring assembly housing) 50
Proximal part of hollow cylinder 51
Distal part of hollow cylinder 52
Sidewall openings of hollow cylinder 53
Open distal end of hollow cylinder 54
Open proximal end (of fluidic conduit) 55
Human heart 60
Left atrium 61
Mitral valve 62
Left ventricle 63
Guidewire 65
Guidewire aperture 66
Proximal end 67 (of fluidic conduit 1)7

## Claims

1. A heart implant system (1) comprising:
a heart implant (2, 20); and
a delivery catheter (3) detachably attached to the heart implant to percutaneously deliver the heart implant to a heart (60) of a subject,
wherein the heart implant comprises an anchoring assembly (4) comprising an anchoring hub (8) and first petals (9) and second petals (10) hingedly attached to the anchoring hub and adjustable relative to the anchoring hub between a crimped delivery configuration and an uncrimped deployed configuration in which the first petals extend radially outwardly of the anchoring hub to limit distal movement of the implant and the second petals form an expandable conforming cage configured to create a stabilising apposition to a wall of a target locus for the implant

2. A heart implant system (1) according to Claim 1, in which the delivery catheter (3) comprises an outer sheath (20) having a distal end (21) configured to receive the anchoring assembly of the heart implant when the anchoring assembly is in a crimped delivery configuration, whereby proximal axial movement of the outer sheath relative to the anchoring assembly effects effect deployment of the first and second petals (9, 10).

3. A heart implant system (1) according to Claim 1, in which the first petals (9) and second petals (10) are configured for self-deployment when the outer sheath (20) is moved proximally relative to the heart implant (2, 20) during deployment.

4. A heart implant system (1) according to Claim 2 or 3, in which the first and second petals (9, 10) are dimensioned for deployment of the first petals before deployment of the second petals.

5. A heart implant system (1) according to any preceding Claim, in which each of the first petals (9) upon deployment assumes a configuration comprising a proximal section (36) that extends radially outwardly of the anchoring hub (8) and a distal section (37) that curves proximally of the proximal section.

6. A heart implant system (1) according to any preceding Claim, in which each of the second petals (10) upon deployment assumes a curved configuration such that the deployed second petals together form a round or oval conforming cage (23).

7. A heart implant system (1) according to any preceding Claim, in which the first and second petals (9, 10) are formed from a shape-set alloy material.

8. A heart implant system according to any preceding Claim, in which each petal comprises a first strut (32, 40), a second strut (32, 40), and a distal apex (35, 43) connecting the first and second struts.

9. A heart implant system (1) according to Claim 8, in which the first and second struts (32) of each first petal (9), upon deployment, have proximal parts that diverge towards a mid-point of the first petal and distal parts that converge away from a mid-point and towards the distal apex (35) of the first petal.

10. A heart implant system (1) according to any preceding Claim, in which the heart implant (2, 30) comprises:
a second assembly (5); and
a connector operably connecting the anchoring assembly (4) and the second assembly (5) in a spaced-apart relationship.

11. A heart implant system (1) according to Claim 10, in which the anchoring assembly (4) is configured for anchoring in a first chamber of the heart and the connector is configured to locate the second assembly (5) in a second chamber of the heart when the anchoring assembly is anchored and stabilised in the first chamber of the heart.

12. A heart implant system (1) according to Claim 11, in which the anchoring assembly (4) is configured for anchoring in a left atrium (61) of the heart and the connector is configured to locate the second assembly (5) in a left ventricle (63) of the heart (60) when the anchoring assembly is anchored in the left atrium.

13. A heart implant system (1) according to any of Claims 10 to 12, in which the second assembly comprises an impeller (13) of a blood pumping device.

14. A heart implant system (1) according to Claim 13, in which the anchoring assembly (4) comprises a motor (7) for the blood pumping device, and the connector comprises a fluidic conduit (17) to deliver blood from the left atrium (61) to the second assembly (5) in the left ventricle (63), wherein the motor and the impeller are operably connected by a torque cable (15).

15. A heart implant system (1) according to any of Claims 12 to 14, in which the anchoring assembly (4) comprises a housing (6) with a fluid inlet and a fluid outlet, the second assembly comprises a housing (12) with a fluid inlet disposed proximally of the impeller (13) and a fluid outlet (14) disposed distally of the impeller, and the connector comprises a fluidic conduit (17) that fluidically connects the fluid outlet of the anchoring assembly and the fluid inlet of the second assembly.
